# EUROPÄISCHE PATENTSCHRIFT

(11) **EP 0 344 580 B1**
(45) Veröffentlichungstag und Bekanntmachung des Hinweises auf die Patenterteilung: **28.12.1994**
(21) Anmeldenummer: 89109261.1
(22) Anmeldetag: 23.05.1989
(51) Int. Cl.: C12Q 1/60

(54) **Verfahren zum Bestimmen der relativen Mengen aller cholesterinhaltigen Lipoproteine in Körperflüssigkeiten**
Method to determine the relative quantities of all cholesterol-containing lipoproteins in body fluids
Méthode pour déterminer les quantités relatives de toutes les lipoprotéines contenant du cholestérol dans les liquides corporels

(30) Priorität: 25.05.1988 DE 3817747
(43) Veröffentlichungstag der Anmeldung: 06.12.1989
(73) Patentinhaber: IMMUNO Aktiengesellschaft, A-1221 Wien (AT)
(72) Erfinder: Aufenanger, Johannes, Dr., D-6945 Hirschberg 2 (DE)
(74) Vertreter: Grünecker, Kinkeldey, Stockmair & Schwanhäusser Anwaltssozietät

(56) Entgegenhaltungen:
- EP-A- 0 183 381
- WO-A-82/00833
- DE-A- 3 640 349
- CHEMICAL ABSTRACTS, Band 102, 1985, Columbus, OH (US); Seite 327, Nr. 163379u#
- CHEMICAL ABSTRACTS, Band 92, 1980, Columbus, OH (US); S. ODA et al., Seite 258, Nr. 211170t#
- CHEMICAL ABSTRACTS, Band 94, 1981, Columbus, OH (US); W. HOHENWALLNER et al., Seite 313, Nr. 60909d#
- CHEMICAL ABSTRACTS, Band 109, 1988, Columbus, OH (US); J. AUFENANGER et al., Seite 421, Nr. 226121w#
- JOURNAL OF ORAL REHABILITATION, Band 13, 1986; Seiten 263-271#
- ANALYTICAL BIOCHEMISTRY, Band 157, 1986; Seiten 32-38#

## Beschreibung

Die Erfindung bezieht sich auf ein Verfahren zum Bestimmen der relativen Mengen aller cholesterinhaltigen Lipoproteine in Körperflüssigkeiten sowie auf ein Verfahren zur Bestimmung der Cholesterinkonzentration dieser Lipoproteine.

Zahlreiche Studien (Lipid Research Clinics Coronary Primary Preventon Trial, 1984, Helsinki Heart Study, 1987, Multiple Risk Faktor Intervention Trial (MRFIT-Studie), 1986, Göttinger Koronarstudie 1980, Kasseler Prospektiv-Studie, seit 1981) belegen den Zusammenhang von Lipoproteinstoffwechselstörungen und Entstehungen von Artherosklerose und Herzinfarkt. In vielen der älteren Studien (Framingham-Studie, 1977) wurde lediglich das HDL (High-Density-Lipoprotein)-Cholesterin als maßgeblicher Faktor zur Entstehung der Atherosklerose betrachtet, von dem angenommen wurde, daß es der empfindlichste Indikator für das Risiko einer koronaren Herzkrankheit für Männer und Frauen von über 50 Jahren sei. Jüngere Studien weisen aber immer deutlicher darauf hin, daß der eigentlich ausschlaggebende Parameter das Verhältnis von LDL (Low-Density-Lipoprotein)-Cholesterin und HDL-Cholesterin ist. Dieses Verhältnis, der sogenannte atherogene Quotient, beschreibt das Verhältnis bzw. Mißverhältnis von Lipoproteinen, die von der Leber als LDL in die Peripherie (Gefäße anderer Organe) gebracht werden und als HDL wieder zur Leber zurücktransportiert werden. Es dient heute als Basis für eine individuelle Risikoabschätzung.

Der Bestimmung von HDL-Cholesterin und LDL-Cholesterin kommt somit bei der Bewertung des individuellen Fettstoffwechselrisikos eine große Bedeutung zu. Im Stand der Technik bemüht man sich daher seit Jahren, schnelle und verläßliche Verfahren zur Trennung und Bestimmung von Cholesterin in den verschiedenen Lipoproteinfraktionen zur Verfügung zu stellen. Das klassische Verfahren der Trennung ist die Ultrazentrifugation. Dieses Verfahren ist jedoch für Routineanalysen nicht brauchbar, weil es erstens sehr zeitraubend ist und zweitens eine kostspielige Ausstattung erfordert. Daraufhin sind Präzipitationsverfahren und elektrophoretische Verfahren für die Trennung der Hauptlipoproteinklassen, nämlich HDL-Cholesterin und LDL-Cholesterin, entsprechend α- und β-Lipoprotein entwickelt worden.

Beispielsweise sind im Stand der Technik elektrophoretisch aufgetrennte Lipoproteine mit Lipid-Färbemitteln, wie z. B. Sudanschwarz B und Fettrot 7B, angefärbt worden; diese Färbemittel färben jedoch unspezifisch alle Lipide und sind nicht spezifisch für Cholesterin oder Cholesterinester.

Spezifischere, neuere Verfahren sehen die Inkubation elektrophoretisch aufgetrennter Lipoproteinfraktionen mit einer Entwicklerlösung vor, die spezifisch mit Cholesterin reagierende Enzyme enthält, beispielsweise Cholesterinesterase und Cholesterinoxidase. So ist aus der DE-OS 28 40 680 ein Verfahren zum Nachweis von Cholesterinlipoproteinen bekannt, in dem die auf einer Celluloseacetatmatrix aufgetrennten Lipoproteine in einer Lösung von Cholesterinesterase und Cholesterinoxidase inkubiert werden. Das Verfahren der DE-OS 28 40 680 führt letztlich zu einem Chinoniminkomplex, der sehr leicht wasserlöslich ist und sehr schnell verblaßt. Darüberhinaus ist das Verfahren wenig präzise, nur bis zu einem Bereich von 1,5 g/l Cholesterin/Fraktion linear und daher vornehmlich zum Nachweis von HDL-Cholesterin brauchbar. Für den Simultannachweis mehrerer Lipoproteinklassen, beispielsweise VLDL (Very-Low-Density-Lipoprotein) LDL und HDL, müssen verschieden verdünnte Proben aufgetragen werden; der Cholesteringehalt von Chylomikronen kann mit diesem Verfahren nicht bestimmt werden.

In Chem. Abstracts, Band 102, 1985, Seite 327, Zusammenfassung Nr. 163379u wird ein Verfahren zum Bestimmen von Lipiden in Lipoproteinfraktionen beschrieben, bei dem die elektrophoretisch aufgetrennten Lipoproteine ebenfalls unter Verwendung von Cholesterinesterase und Cholesterinoxidase angefärbt werden. Das beschriebene Verfahren scheint weitgehend mit dem zuvor diskutierten, in der DE-OS-2840680 offenbarten Verfahren identisch zu sein, und ist mit den gleichen Nachteilen verbunden.

In Chem. Abstracts, Band 92, 1980, Seite 258, Zusammenfassung Nr. 211170t wird ein Verfahren zum Bestimmen von HDL-Cholesterin offenbart. Das beschriebene Verfahren bedient sich der Dünnschichtelektrophorese zum Auftrennen der Lipoproteinfraktionen sowie des Enzymsystems Cholesterinesterase/Cholesterinoxidase zum Nachweis des gebundenen Cholesterins. Das Verfahren dient jedoch nur zur Bestimmung der absoluten Menge vorhandenen HDL's; aus der Originalpublikation geht weiter hervor, daß eine Auftrennung der LDL- und VLDL-Banden mit diesem Verfahren nicht möglich ist.

In der japanischen Patentoffenlegungsschrift 58-210000 (1983) wird ein Verfahren beschrieben, das den Nachweis von Cholesterin unter Verwendung von Cholesterinesterase und Cholesterindehydrogenase vorsieht; die dabei entstehenden wasserunlöslichen Formazankomplexe werden in einem Gel von über 1 mm Dicke gebildet. Das in dieser Anmeldung vorgeschlagene Nachweissystem erfordert sehr lange Elektrophoresezeiten und Entwicklungszeiten und ist aufgrund eines sehr hohen Bedarfs an teuren Enzymen sehr kostspielig und für Routine-Untersuchungen unbrauchbar; weiterhin sind die Ergebnisse äußerst schlecht archivierbar.

In der nach dem Prioritötstag der gegenwärtigen Anmeldung veröffentlichten deutschen Patentanmeldung DE-A 36 40 349 ist ein Verfahren beschrieben, das es gestattet, die mit dem in der genannten japanischen Patentoffenlegungsschrift beschriebenen Verfahren verbundenen Kosten stark herabzusetzen. Die Geschwindigkeit und die Archivierbarkeit scheint jedoch verbesserungswürdig.

Der vorliegenden Erfindung lag daher die Aufgabe zugrunde, ein Verfahren zum Bestimmen der relativen Mengen aller cholesterinhaltigen Lipoproteine in Körperflüssigkeiten, wobei die Lipoproteine eines Aliquotes der Körperflüssigkelt auf einer Trägermatrix elektrophoretisch aufgetrennt werden und anschließend durch eine enzymatische Behandlung, die eine Inkubation der Trägermatrix mit den Enzymen Cholesterinesterase und Cholesterindehydrogenase sowie dem Coenzym Nicotinamid-Adenin-Dinucleotid umfaßt und zur Bildung eines nachweisbaren Formazankomplexes führt, nachgewiesen werden und die relativen Mengen der verschiedenen Lipoproteinklassen bestimmt werden, zu entwickeln, das eine schnelle und zuverlässige Bestimmung aller diagnostisch relevanten Lipoproteinfraktionen innerhalb kurzer Zeit erlaubt, wobei das Verfahren zusätzlich reproduzierbar und das Ergebnis archivierbar sein sollten.

Diese Aufgabe wird in einem Verfahren der zuvor genannten Art dadurch gelöst, daß die Elektrophorese auf einer Dünnschichtmatrix mit einer Dicke von 0,1 mm bis 0,5 mm durchgeführt wird.

Mit dem erfindungsgemäßen Verfahren ist es möglich, innerhalb sehr kurzer Zeit die relativen Mengen aller Lipoproteinfraktionen aus beliebigen Körperflüssigkeiten, beispielsweise Plasma oder Serum, zuverlässig zu bestimmen und sogar Proben aus heparinisierten Patienten zuverlässig analysieren zu können. Die dem erfindungsgemäßen Verfahren zugrundeliegende Reaktion sieht dabei die Dehydrogenierung des aus dem Lipoprotein freigesetzten Cholesterins zum Δ ⁵-Cholesten-3-on vor, indem Elektronen vom Cholesterin via NAD⁺/NADH+H⁺ auf einen Elektronenkoppler, beispielsweise PMS, und von dort auf einen letzten Elektronenakzeptor, bevorzugt ein Tetrazoliumsalz, übertragen werden. Diese Reaktionskette führt zur Bildung eines stabilen, wasserunlöslichen Formazankomplexes, der in der Dünnschichtmatrix densitometrisch bestimmt werden kann. Der atherogene Quotient, d.h., das Verhältnis von LDL-Cholesterin zu HDL-Cholesterin, kann anschließend direkt aus den densitometrischen Flächenintegralen der beiden Lipoproteinfraktionen gebildet werden. Dabei war es höchst überraschend, bei der Verwendung der Dünnschichtmatrizes auch bei Auftrennung von nur einem µl der jeweiligen Körperflüssigkeit durchaus ausreichende Mengen gebildeter Komplexe an den jeweiligen Positionen der Matrix vorzufinden. Dies war deshalb überraschend, weil der Fachmann erwartet hätte, eine für die Bildung des Formazankomplexes unzureichende Menge des primären Elektronendonors, nämlich des Cholesterins, in der Dünnschichtmatrix vorliegen zu haben.

Mit dem erfindungsgemäßen Verfahren lassen sich jedoch überraschenderweise äußerst geringe Mengen Cholesterin zuverlässig bestimmen: Die untere Nachweisgrenze liegt bei nur 5 ng pro Spur und Bande. Mit dem erfindungsgemäßen Verfahren lassen sich erstmals HDL-, LDL-, VLDL- und Lp(X)-Cholesterin nebeneinander und mit hoher Präzision bestimmen. Die Cholesterinkonzentrationen sind im Bereich von 50-3200 mg/l Körperflüssigkeit exakt bestimmbar.

Der im erfindungsgemäßen Verfahren gebildete Formazankomplex ist äußerst schwer wasserlöslich. Dies hat den Vorteil, daß auch in einer relativ wenig konzentrierten Matrix ein bereits gebildeter Komplex wenig diffundiert und die resultierenden Banden daher sehr scharf begrenzt sind. Darüberhinaus ist der gebildete Formazankomplex sehr lichtbeständig und daher auch nach geraumer Zeit noch mit hoher Reproduzierbarkeit und mit gleicher Intensität nachweisbar. Es hat sich gezeigt, daß selbst bei hohen Cholesterinkonzentrationen der für die Elektrophorese verwendeten Körperflüssigkeit der gebildete Formazankomplex bei der Trocknung der Dünnschichtmatrix nicht auskristallisiert, sondern auch in der getrockneten Matrix zu einer scharfen, densitometrisch nach wie vor sehr gut auswertbaren Bande führt.

Für die Durchführung des erfindungsgemäßen Verfahrens können Trägermatrizes aller Art verwendet werden, bevorzugt aber Agarose oder Polyacrylamid. Beide Matrixmaterialien sind in für das erfindungsgemäße Verfahren ausreichender Qualität auf dem Markt. An die verwendete Matrix ist lediglich die Anforderung zu stellen, daß sie frei von Stabilisatoren und Enzyminhibitoren ist, die die im Anschluß an die Elektrophorese stattfindende enzymatische Umsetzung gefährden könnten. Die Matrix sollte darüberhinaus für Dünnschichtelektrophorese brauchbar sein, d. h. eine einigermaßen stabile Matrix in der gewünschten Dicke bilden können. Die Probenapplikation auf die Matrix erfolgt beispielsweise mit Hilfe einer Schlitzfolie. Das dabei aufgetragene Probenvolumen beträgt bevorzugt ca. 5 µl und diffundiert innerhalb einer akzeptablen Zeit von ca. 10 min in die Matrix. In Abhängigkeit von der Schlitzabmessungen und der Zeit kann das Probenvolumen selbstverständlich auch vergrößert oder verringert werden.

In einer bevorzugten Ausführungsform wird als Matrix ein 0,8 bis 1,2 gew.-%iges Agarosegel in einem geeigneten Puffer verwendet. Dieses Gel sollte eine Dicke von bevorzugt 0,35 mm aufweisen.

Dem Gel werden bis zu 0,5 Gew.-% Albumin beigemischt, um die Homogenität der Banden zu fördern. Es können sowohl Humanserumalbumin- als auch Rinderserumalbuminpräparationen oder andere verwendet werden. Die Verwendung von Albumin ist nicht essentiell, macht jedoch die Banden homogener, was zu einer einfachen quantitativen Auswertung führt.

Das erfindungsgemäße Verfahren sieht vor, daß die Dünnschichtmatrix nach der Elektrophorese mit einer Entwicklerlösung in Kontakt gebracht wird, durch deren Einwirkung auf das Gel die cholesterinhaltigen Lipoproteinbanden der jeweils elektrophoretisch aufgetrennten Flüssigkeit nachgewiesen werden können. Diese Entwicklerlösung enthält neben den beiden Enzymen Cholesterinesterase und Cholesterindehydrogenase das Coenzym Nicotinamid-Adenin-Dinucleotid der Cholesterindehydrogenase, einen Elektronenkoppler und einen Farbindikator. Dabei bewirkt die Cholesterinesterase eine Abspaltung des Cholesterins aus dem Cholesterinester im jeweiligen Lipoprotein, die Cholesterindehydrogenase eine Dehydrogenierung zum Cholesten-3-on, der Elektronenkoppler nimmt die bei der Dehydrogenierung des Cholesterins in Form eines Hydridions auf das Nicotinamid-Adenin-Dinucleotid übertragenen Elektronen auf und oxidiert das Coenzym dadurch wieder, und letztlich führt der Transfer der Elektronen auf einen geeigneten Farbindikator zur Bildung eines nachweisbaren Farbkomplexes. Die Entwicklerlösung enthält in der Regel übliche Zusätze, beispielsweise puffernde Substanzen, um die Reaktion im Bereich des pH-Optimums der beteiligten Enzyme durchführen zu können, sowie gegebenenfalls einen Chelatbildner, der durch Komplexierung gegebenenfalls anwesender Metallionen, beispielsweise Mg²⁺ oder Zn²⁺, von der Gegenwart dieser Metallionen abhängige Proteasen inhibiert.

Bevorzugte Puffersysteme sind in diesem Zusammenhang Tris(hydroxymethyl)aminomethan (Tris), Barbital oder eine Mischung davon. Ein bevorzugt einzustellender pH-Bereich liegt zwischen 7,8 und 8,6.

Bei der Auswahl eines geeigneten Elektronenkopplers zur Durchführung des erfindungsgemäßen Verfahrens bieten sich zwei grundsätzlich verschiedene Möglichkeiten an. Aus der japanischen Patentoffenlegungsschrift 58-210000 (1983) ist bereits die Verwendung eines enzymatischen Elektronenkopplers, nämlich des Enzymes Diaphorase bekannt. Dadurch wird jedoch eine weitere biologische Substanz in das Verfahren eingeführt, die wie jedes Enzym eine geringere Stabilität und größere Empfindlichkeit als eine organisch chemische Substanz aufweist. Bevorzugt wird daher als Elektronenkoppler Phenazinmethosulfat (PMS) verwendet.

Der durch die Reakton letztendlich reduzierte Farbindikator ist erfindungsgemäß ein Tetrazoliumsalz, bevorzugt Nitroblautetrazoliumchlorid (NTB) oder 2-(p-Jodphenyl)-3-(p-nitrophenyl)-5-phenyltetrazoliumchlorid (INT). Prinzipiell ist jedes Tetrazoliumsalz verwendbar, es sollte jedoch bevorzugt einen hohen molaren Extinktionskoeffizienten aufweisen, um die Empfindlichkeit des erfindungsgemäßen Verfahrens nicht zu gefährden.

In einer bevorzugten Ausführungsform des erfindungsgemäßen Verfahrens enthält die Entwicklerlösung die folgenden Substanzen:

| | | |
|---|---|---|
| Tris-HCl-Puffer: | 30 - 60 mM, | bevorzugt 57 mM |
| NAD: | 0,2 - 2,0 mM, | bevorzugt 0,5 mM |
| EDTA: | 0,05 - 0,2 mM, | bevorzugt 0,1 mM |
| NTB oder INT: | 0,05 - 3,0 mM, | bevorzugt 0,16 mM |
| PMS: | 0,01 - 0,05 mM, | bevorzugt 0,03 mM |
| bei einem pH von: | 7,8 - 8,6, | bevorzugt pH 8,0, |
| Cholesterinesterase: | 0,2 - 1,0 U/ml, | bevorzugt 0,4 U/ml |
| Cholesterinedehydrogenase: | 0,07 - 1,0 U/ml, | bevorzugt 0,14 U/ml |

Bei Verwendung der aufgeführten Substanzen in den angegebenen Konzentrationsbereichen erreicht das erfindungsgemäße Verfahren seine höchste Empfindlichkeit.

Die in einer bevorzusten Ausführungsform des erfindungsgemäßen Verfahren verwendeten Konzentrationen der einzelnen Bestandteile, insbesondere der verwendeten Enzyme, liegt deutlich unter den in der japanischen Patentoffenlegungsschrift 58-210000 (1983) angegebenen. Dies ist zu einem ganz wesentlichen Teil auf die Verwendung der Dünnschichtmatrix zurückzuführen, in der die Lipoprotein-Cholesterinmoleküle durch Diffusion besser erreicht werden können.

Die Entwicklung der Dünnschichtmatrix in der Entwicklerlösung kann entweder bei 37 ° C ca. 45 min erfolgen, ebenso jedoch auch bei Raumtemperatur durchgeführt werden. Eine Entwicklung bei Raumtemperatur verläuft etwas langsamer, in diesem Fall sollten der Reaktion 2 h Zeit gegeben werden.

Ein besonderer Vorteil des erfindungsgemäßen Verfahrens ist es, daß die entwickelte Dünnschichtmatrix nach im Stand der Technik bekannten Verfahren getrocknet werden kann. Die Matrix, die im Zusammenhang z. B. mit einer Krankenakte dokumentarischen Charakter hat, ist dadurch zum einen besser handhabbar und damit leichter auswertbar, zum anderen bequem archivierbar. Diese Eigenschaften stellen einen ganz wesentlichen Vorteil des erfindungsgemäßen Verfahrens über die Verfahren aus dem Stand der Technik dar.

Die Auswertung der entwickelten Dünnschichtmatrix erfolgt nach ebenfalls bekannte Verfahren z. B. densitometrisch. Die für die Densitometrie verwendete Wellenlänge hängt dabei von der Wahl des letzten Elektronenakzeptors ab und beträgt beispielsweise im Fall der Verwendung von INT 570 nm, im Fall der Verwendung von NTB 546 nm. Es ist dabei offensichtlich, daß für jedes alternativ verwendete Tetrazoliumsalz die Wellenlänge seines Absorptionsmaximums für die Densitometrie herangezogen wird.

Erfindungsgemäß wird zusätzlich zum Verfahren zur Bestimmung der relativen Mengen aller cholesterinhaltigen Lipoproteine in Körperflüssigkeiten ein Verfahren zum Bestimmen der Cholesterinkonzentration aller cholesterinhaltigen Lipoproteine in Körperflüssigkeiten zur Verfügung gestellt. Dieses Verfahren sieht zusätzlich zum bereits beschriebenen Verfahren vor, daß der Cholesteringesamtgehalt der jeweiligen Probe nach im Stand der Technik bekannten Verfahren bestimmt wird und die durch densitometrische Auswertung erhaltenen relativen Anteile der verschiedenen cholesterinhaltigen Lipoproteine auf diese Gesamtmenge bezogen werden. Auf diese Art und Weise können die Absolutwerte der Konzentrationen der verschiedenen Lipoproteinfraktionen in den Körperflüssigkeiten eines Patienten bestimmt werden.

Das folgende Beispiel erläutert die Erfindung:

### Beispiel 1

### Herstellen der Dünnschichtagarose-Gele

0,1 g Agarose (Standard EEO (Elektroendoosmose)) wurden gut in 10 ml Barbital-Glycin-Puffer (50 mmol/l, pH8,6) suspendiert und im Rückflußkühler 30 min lang gekocht. Nach dem Abkühlen der Lösung auf 60 ° C wurden 0,02 g Albumin in ihr gelöst.

Für die Herstellung eines Geles wurden 2 ml dieser Agaroselösung auf einem erwärmten Nivelliertisch (60 ° C) auf vorbereitete Gelträgerfolien (Gelfond^{R}, Serva, -Heidelberg; 7 x 8 cm) mittels einer vorgewärmten Pipette aufgebracht und gleichmäßig verteilt. Die Agarose wurde sodann unter staubfreien Bedingungen erkalten und gelieren gelassen. Nach dem Gelieren wurde das Gel mit einer Folie abgedeckt und vor dem Gebrauch eine Nacht im Kühlschrank durchhärten gelassen.

### Durchführung der Elektrophorese

Die Schutzfolie wurde vom Gel entfernt und das Gel auf eine horizontale Unterlage gelegt. Überschüssige Feuchtigkeit wurde von der Geloberfläche durch Auflegen eines Gelblotter (Filterpapier) entfernt. Anschließend wurde eine Probenmaske (Schlitzmaske mit 6 Schlitzen 8 x 0,5 mm) im ersten Drittel des Geles aufgelegt und diese leicht angedrückt, so daß unter der Maske keine Luftblasen mehr zu sehen waren.

Auf die Probenschlitze wurden je 5 µl Serum verschiedener Patienten aufgetragen und 10 min ins Gel eindiffundieren gelassen. Nach 10 min Diffusionszeit nicht eindiffundierte Probe wurde mit dem Probenblotter (Filterpapierstreifen) aufgesaugt und die Schlitzmaske anschließend vorsichtig entfernt.

Das Gel wurde dann in einen Folienhalter so eingespannt, daß die Gelschicht nach unten wies. Die Folie stellte sich in Form eines Rundbogens dar.

Der Folienhalter mit dem eingespannten Gel wurde auf den Elektrodenträger der mit dem Elektrodenpuffer (siehe unten) gefüllten Elektrophoresekammer, gesetzt. Die Gelenden ragten dabei ca. 0,5 cm in den Puffer hinein.

Die Proben wurden durch 40minütige Elektrophorese (90 V) aufgetrennt.

Elektrodenpuffer für die Elektrophorese:
1 l Barbital-Glycin-Puffer (50 mmol/l, pH 8,6)

### Durchführen der enzymatischen Behandlung

Für die enzymatische Behandlung wurden folgende Stammlösungen hergestellt:
I. Puffer A (Modifizierter Tris-Puffer, 0,3 M)
   18,2 g Trishydroxymethylaminomethan in 400 ml aq.bidest auflösen und mit 1 N HCl auf pH 8,0 einstellen; 4 ml Triton® X-100 und 0,5 ml DMSO (Dimethylsulfoxid) hinzugeben, sodann auf 500 ml mit aq.bidest auffüllen.
II. Phenazinmethosulfat (PMS), 3,27 mM
   100 mg PMS in 100 ml aq.bidest lösen.
III. Nitroblau-Tetrazolium-Chlorid (NTB)
   100 mg NTB in 100 ml aq.bidest lösen.
IV. Nicotin-adenin-dinucleotid (NAD), 13 mM
   1000 mg NAD in 10 ml verdünnten Puffer A (1:4 mit aq.bidest verdünnt) lösen.
V. Ethylendiamintetraessigsäure-dihydrat (Dinatrium-Salz), 10 mM
   372 mg EDTA*2H₂O in 100 ml aq.bidest lösen.
VI. Cholesterin-Dehydrogenase (45,2 U/ml)
VII. Cholesterin-Esterase 52 U/ml
Kurz vor Ablauf der Elektrophorese wurde das Reaktionsgemisch wie folgt angesetzt:
In eine Färbeküvette (Größe 7,5 x 8,5 cm, lichtundurchlässig) wurden aus den Stammlösungen pipettiert:

| | |
|---|---|
| Puffer A, pH 8,0 | 1,25 ml |
| NAD | 0,25 ml |
| EDTA | 0,06 ml |
| NTB | 0,60 ml |
| PMS | 0,06 ml |
| Cholesterin-Dehydrogenase | 0,02 ml |
| Cholesterin-Esterase | 0,05 ml |
| Aqua bidest | 4,4 ml |

Nach Beendigung der Elektrophorese wurde die Gelfolie in die Färbeküvette gelegt und mittels einer "Gel-Schaukel" unter leichten Bewegungen 2 h bei Raumtemperatur inkubiert.

Nach der Inkubation wurde das Gel aus dem Reaktionsgemisch genommen und von beiden Seiten mit aq. dest. abgespült. Die violette Farbe der cholesterinhaltigen Banden war deutlich sichtbar. Das Gel wies mehrere scharfe, kräftige Banden auf.

Die Unterseite der Folie wurde mit einem weichen Zellstofftuch getrocknet und jede einzelne Bahn bei 546 nm im Geldensitometer densitometriert. Das Gel wurde nach der Auswertung zum Zwecke der Archivierung für 30 min in 1 %ige Essigsäure gelegt (Auswaschen der photoempfindlichen Reste von PMS und NTB) und anschließend staubfrei bei Raumtemperatur getrocknet. Eine zu Vergleichszwecken durchgeführte densitometrische Bestimmung der Banden nach dem Trocknen ergab keine wesentlichen Abweichungen von den zuvor erzielten Ergebnissen.

### Quantifizierung des Cholesteringehaltes in den Lipoproteinbanden

Der Cholesteringehalt der einzelnen Lipoproteinfraktionen wurde nach Bestimmung des Gesamtcholesteringehaltes der aufgetragenen Proben (enzymatische Cholesterinbestimmung mittels Cholesterinoxidase-Peroxidase-Methode) bestimmt. So wurden für einen der Patienten folgende Werte festgestellt:

| | |
|---|---|
| Gesamtcholesteringehalt im Serum: | 200 mg/dl |

| Densitometerausdruck: | |
|---|---|
| β-Lipoprotein(LDL)-Cholesterinbande: | 60 % |
| prä-β-Lipoprotein(VLDL)-Cholesterinbande: | 10 % |
| α-Lipoprotein(HDL)-Cholesterinbande: | 30 % |

| Daraus folgt: | |
|---|---|
| LDL-Cholesterin | 120 mg/dl |
| VLDL-Cholesterin | 20 mg/dl |
| HDL-Cholesterin | 60 mg/dl |

## Patentansprüche

1. Verfahren zum Bestimmen der relativen Mengen aller cholesterinhaltigen Lipoproteine in Körperflüssigkeiten, wobei die Lipoproteine eines Aliquotes der Körperflüssigkeit auf einer Trägermatrix elektrophoretisch aufgetrennt werden und anschließend durch eine enzymatische Behandlung, die eine Inkubation der Trägermatrix mit den Enzymen Cholesterinesterase und Cholesterindehydrogenase sowie dem Coenzym Nicotinamid-Adenin-Dinucleotid umfaßt und zur Bildung eines nachweisbaren Formazankomplexes führt, nachgewiesen werden und die relativen Mengen der verschiedenen Lipoproteinklassen bestimmt werden, **dadurch gekennzeichnet**, daß die Elektrophorese auf einer Dünnschichtmatrix mit einer Dicke von 0,1 bis 0,5 mm durchgeführt wird.

2. Verfahren nach Anspruch 1, **dadurch gekennzeichnet**, daß das Matrixmaterial der Dünnschichtmatrix Polyacrylamid oder Agarose umfaßt.

3. Verfahren nach mindestens einem der Ansprüche 1 bis 2, **dadurch gekennzeichnet**, daß die Dünnschichtmatrix zusätzlich bis zu 0,5 Gew.-% Albumin enthält.

4. Verfahren nach mindestens einem der Ansprüche 1 bis 3, **dadurch gekennzeichnet**, daß die Dünnschichtmatrix nach der Elektrophorese mit einer Entwicklerlösung in Kontakt gebracht wird, die Cholesterinesterase, Cholesterindehydrogenase, Nikotinamid-Adenin-Dinucleotid (NAD), einen Elektronenkoppler und einen Farbindikator sowie gegebenenfalls übliche Zusätze, beispielsweise puffernde Substanzen und Chelatbildner, enthält.

5. Verfahren nach Anspruch 4, **dadurch gekennzeichnet**, daß als puffernde Substanz Tris(hydroxymethyl)aminomethan (Tris), Barbital oder eine Mischung davon verwendet wird.

6. Verfahren nach Anspruch 4 oder 5, **dadurch gekennzeichnet**, daß der Elektronenkoppler Phenazinmethosulfat (PMS) oder Diaphorase ist.

7. Verfahren nach mindestens einem der Ansprüche 4 bis 6, **dadurch gekennzeichnet**, daß der Farbindikator ein Tetrazoliumsalz, bevorzugt Nitroblautetrazoliumchlorid (NTB) oder 2-(p-Jodphenyl)-3-(p-nitrophenyl)-5-phenyltetrazoliumchlorid (INT), ist.

8. Verfahren nach mindestens einem der Ansprüche 4 bis 7, **dadurch gekennzeichnet**, daß die Entwicklerlösung die folgenden Substanzen enthält: Tris-HCl-Puffer, pH 7,8-8,6, bevorzugt pH 8,0: 30-60 mM, bevorzugt 57 mM; NAD: 0,2-2,0 mM, bevorzugt 0,5 mM; EDTA: 0,05-0,2 mM, bevorzugt 0,1 mM; NTB oder INT: 0,05-3,0 mM, bevorzugt 0,16 mM; PMS: 0,01-0,05 mM, bevorzugt 0,03 mM; Cholesterindehydrogenase: 0,07-1,0 U/ml, bevorzugt 0,14 U/ml; Cholesterinesterase: 0,2-2,0 U/ml, bevorzugt 0,4 U/ml.

9. Verfahren nach mindestens einem der Ansprüche 1 bis 8, **dadurch gekennzeichnet**, daß die entwickelte Dünnschichtmatrix getrocknet wird.

10. Verfahren nach mindestens einem der Ansprüche 1 bis 9, **dadurch gekennzeichnet**, daß die entwickelte Dünnschichtmatrix densitometriert wird.

11. Verfahren nach einem der Ansprüche 1 bis 10, **dadurch gekennzeichnet**, daß die densitometrische Auswertung im Fall der Verwendung von INT bei 570 nm, im Fall der Verwendung von NTB bei 546 nm durchgeführt wird.

12. Verfahren zum Bestimmen der Konzentration aller cholesterinhaltigen Lipoproteine in Körperflüssigkeiten, wobei die mit dem Verfahren nach einem der Ansprüche 1 bis 11 ermittelten relativen Mengen der cholesterinhaltigen Lipoproteine im Verhältnis zu der Gesamtcholesterinkonzentration der Körperflüssigkeit bestimmt werden.

## Claims

1. Process for the determination of the relative quantities of all lipoproteins containing cholesterol in body fluids, wherein the lipoproteins of an aliquot of the body fluid are electrophoretically separated on a supporting matrix and are then detected by an enzymatic treatment which comprises incubation of the supporting matrix with the enzymes cholesterol esterase and cholesterol dehydrogenase together with the co-enzyme nicotinamide-adenine dinucleotide and leads to the formation of a detectable formazan complex and the relative quantities of the various classes of lipoproteins are determined, characterised in that electrophoresis is performed on a thin layer matrix with a thickness of 0.1 to 0.5 mm.

2. Process according to claim 1, characterised in that the matrix material of the thin layer matrix comprises polyacrylamide or agarose.

3. Process according to at least one of claims 1 to 2, characterised in that the thin layer matrix additionally contains up to 0.5 wt.% albumin.

4. Process according to at least one of claims 1 to 3, characterised in that, after electrophoresis, the thin layer matrix is brought into contact with a developer solution which contains cholesterol esterase, cholesterol dehydrogenase, nicotinamide-adenine dinucleotide (NAD), an electron coupler and a colour indicator, optionally together with customary additives, for example buffering substances and chelating agents.

5. Process according to claim 4, characterised in that the buffering substance used is tris(hydroxymethyl)aminomethane (tris), barbital or a mixture thereof.

6. Process according to claim 4 or 5, characterised in that the electron coupler is phenazine methosulphate (PMS) or diaphorase.

7. Process according to at least one of claims 4 to 6, characterised in that the colour indicator is a tetrazolium salt, preferably nitro blue tetrazolium chloride (NTB) or 2-(*p*-iodophenyl)-3-(*p*-nitrophenyl)-5-phenyltetrazolium chloride (INT).

8. Process according to at least one of claims 4 to 7, characterised in that the developer solution contains the following substances: tris-HCl buffer, pH 7.8-8.6, preferably pH 8.0: 30-60 mM, preferably 57 mM; NAD: 0.2-2.0 mM, preferably 0.5 mM; EDTA: 0.05-0.2 mM, preferably 0.1 mM; NTB or INT: 0.05-3.0 mM, preferably 0.16 mM; PMS: 0.01-0.05 mM, preferably 0.03 mM; cholesterol dehydrogenase: 0.07-1.0 U/ml, preferably 0.14 U/ml; cholesterol esterase: 0.2-2.0 U/ml, preferably 0.4 U/ml.

9. Process according to at least one of claims 1 to 8, characterised in that the developed thin layer matrix is dried.

10. Process according to at least one of claims 1 to 9, characterised in that the developed thin layer matrix is densitometrically evaluated.

11. Process according to one of claims 1 to 10, characterised in that the densitometric evaluation is performed at 570 nm if INT is used, at 546 nm if NTB is used.

12. Process for the determination of the concentration of all lipoproteins containing cholesterol in body fluids, wherein the relative quantities of lipoproteins containing cholesterol determined using the process according to one of claims 1 to 11 are determined in relation to the total cholesterol concentration of the body fluid.

## Revendications

1. Procédé de détermination de la quantite relative de toutes les lipoprotéines contenant du cholestérol présentés dans les liquides de l'organisme, procédé selon lequel les lipoprotéines d'une partie aliquote du liquide organique sont résolues par électrophorèse dans une matrice support, puis mises en évidence par un traitement enzymatique qui comprend une incubation de la matrice support avec les enzymes cholestérolestérase et cholestéroldeshydrogénase, ainsi qu'avec la coenzyme nicotinamide-adenine-dinucleotide et qui conduit a la formation d'un complexe de formazan, procédé selon lequel les quantités relatives des diverses classes de lipoprotéines sont déterminées et qui est caractérisé en ce que l'électrophorèse est effectuée dans une matrice formant une couche mince dont l'épaisseur est comprise entre 0,1 et 0,5 mm.

2. Procédé selon la revendication 1, caractérisé en ce que la substance de la matrice formant une couche mince comprend du polyacrylamide ou de l'agarose.

3. Procédé selon l'une au moins des revendications 1 et 2, caractérisé en ce que la matrice formant une couche mince contient en plus jusqu'à 0,5% en poids d'albumine.

4. Procédé selon l'une au moins des revendications 1 à 3, caractérisé en ce que la matrice formant une chouche mince est mise en contact, après l'électrophorèse, avec une solution révélatrice, qui contient de la cholestérolestérase, de la cholestéroldeshydrogénase, de la nicotinamide-adénine-dinucléotide (NAD), un agent de couplage électronique et un indicateur coloré, ainsi que, si besoin est, les additifs habituels comme par exemple les tampons et les chélateurs.

5. Procédé selon la revendication 4, caractérisé en ce que le tris(hydroxymethyl)aminométhane (Tris), le barbital ou un mélange des deux est utilisé comme tampon.

6. Procédé selon la revendication 4 ou 5, caractérisé en ce que l'agent de couplage électronique est le methosulfate de phénazine (PMS) ou la diaphorase.

7. Procédé selon l'une au moins des revendications 4 à 6, caractérisé en ce que l'indicateur coloré est un sel de tetrazolium, de préférence le chlorure de bleu de nitrotétrazolium (NTB) ou le chlorure de 2-(p-iodophényl)-3-(p-nitrophényl)-5--phényltetrazolium (INT).

8. Procédé selon l'une au moins des revendications 4 à 7, caractérisé en ce que la solution révélatrice contient les substances suivantes: 30-60 mM, de préférence 57 mM, de tampon Tris-HCl, pH 7,8-8,6, de préférence pH 8,0, 0,2-2,0 mM, de préférence 0,5 mM, de NAD, 0,05-0,2 mM, de préférence 0,1 mM, d'EDTA, 0,05-3,0 mM, de préférence 0,16 mM, de NTB ou d'INT, 0,01-0,05, de préférence 0,03 mM, de PMS, 0,07-1,0 U/ml, de préférence 0,14 U/ml, de cholestéroldeshydrogénase, 0,2-2,0 U/ml, de préférence 0,4 U/ml, de cholestérolestérase.

9. Procédé selon l'une au moins des revendications 1 à 8, caractérisé en ce que la matrice formant une couche mince est séchée une fois que son contenu a été révélé.

10. Procédé selon l'une au moins des revendications 1 a 9, caractérisé en ce que la matrice formant une couche mince est soumise a une évaluation densitométrique une fois que son contenu a été révélé.

11. Procédé selon l'une quelconque des revendications 1 à 10, caractérisé en ce que l'évaluation densitométrique est effectuée à 570 nm, si de l'INT a été utilisé, et à 546 nm, si du NTB a été utilisé.

12. Procédé de détermination de la concentration de toutes les lipoprotéines contenant du cholestérol présentes dans les liquides de l'organisme, procédé selon lequel les quantités relatives de lipoprotéines contenant du cholestérol, quantités qui doivent être déterminées par le procédé selon l'une quelconque des revendications 1 à 11, sont évaluées proportionnellement à la teneur totale en cholestérol du liquide de l'organisme.
